# EUROPEAN PATENT APPLICATION

(11) **EP 4 372 755 A1**
(43) Date of publication of application: **22.05.2024**
(21) Application number: 22209360.1
(22) Date of filing: 24.11.2022
(51) Int. Cl.: G16H 30/00, G06F 3/033, G06F 3/04812

(54) **INPUT DEVICE RESTRICTION MANAGEMENT FOR SAFE REMOTE OPERATION OF MEDICAL DEVICES**

(30) Priority: 16.11.2022 US 202263425697 P
(71) Applicant: Koninklijke Philips N.V., 5656 AG Eindhoven (NL)
(72) Inventor: AMTHOR, Thomas Erik, Eindhoven (NL)
(74) Representative: Philips Intellectual Property & Standards

(57) **Abstract**

An assistive system (1) including an assistor electronic device (12) including an assistor display (24), and at least a pointing device (22), the assistor electronic device programmed to perform a controller mirror task including receiving and displaying a mirror of the controller video on the assistor display and receiving remote inputs directed to the electronic imaging device controller via the at least one user input device of the assistor electronic device; and assistor electronic device interfacing hardware (40) configured to transmit the controller video from the electronic imaging device controller to the assistor electronic device and including a human interface device (HID) restriction processor (46) configured to receive the remote inputs from the assistor electronic device; transmit those remote inputs that satisfy an access criterion (51) to the electronic imaging device controller; and block those remote inputs that do not satisfy the access criterion from the electronic imaging device controller.

## Description

### FIELD OF THE INVENTION

The following relates generally to the imaging arts, remote imaging assistance arts, remote imaging examination monitoring arts, and related arts.

### BACKGROUND OF THE INVENTION

Medical imaging, such as computed tomography (CT) imaging, magnetic resonance imaging (MRI), positron emission tomography (PET) imaging, fluoroscopy imaging, and so forth, is a critical component of providing medical care, and is used in a wide range of medical fields, such as cardiology, oncology, neurology, orthopedics, to name a few. The operator of the medical imaging device used to acquire the medical images is typically a trained imaging technologist, while interpretation of the medical images is often handled by a medical specialist such as a radiologist.

Currently, medical imaging is in high demand. As the world population ages, the demand for quick, safe, high quality medical imaging will only continue to grow, putting further pressure on imaging centers and their staff. Under such conditions, errors can occur, and can often be costly. One approach for imaging centers to boost efficiency and grow operations at no extra labor costs is through a radiology operations command center (ROCC) system. Radiology operations command centers enable teams to work across the entire network of imaging sites, providing their expertise as needed and remotely assisting less experienced technologists in carrying out high quality scans. Remote technologists or experts can monitor the local operators of scanning procedures through cameras installed in the scanning areas (or from other sources, such as sensors (including radar sensors), console video feeds, microphones connected to Internet of Things (IoT) devices, and so forth. In addition, these sources can be supplemented by other data sources like Health-Level 7 (HL7), Digital Imaging and Communications in Medicine (DICOM), Electronic Health Record (EHR) databases, and so forth.

While communication and screen sharing capabilities generally do not implicate regulatory concerns in such ROCC systems, the integration of a real time remote control of imaging systems is more challenging from the regulatory perspective. Remote connection of universal serial bus (USB) devices, such as a mouse or keyboard, theoretically allow a remote user to take control of devices interacting with patients in a sensitive and highly regulated environment. These Human Interface Devices (HID) or Physical Interface Devices (PID) are typically connected via a standardized USB protocol that defines messages for keystroke or mouse movement events. Such real time remote control can be useful in an ROCC context, for example by permitting a remote expert to use the on-screen pointer of the mouse or other pointing device to point out features on the controller display, or enabling the remote expert to directly modify certain scan settings rather than trying to explain to the local imaging technologist how to do so.

However, deployment of such real time remote control of a medical imaging device can raise patient privacy and safety considerations. For example, unsupervised or unintended triggering of any interaction of the medical device engaged with a patient may put the patient at risk. In addition, remote operation of a medical device may enable a remote operator to access and view sensitive patient data that would not be required to access or view for the intended task. Moreover, remote operation capabilities of any computing device in the hospital comes with the risk of malicious attack (hacking), where an intruder would try to impact the operation of a medical device or to get access to sensitive information or even surreptitiously access and potentially maliciously attack the supporting hospital information technology (IT) network.

The following discloses certain improvements to overcome these problems and others.

### SUMMARY OF THE INVENTION

In one aspect, an assistive system for providing remote assistance to a local operator of a medical imaging device having an electronic imaging device controller and a controller display presenting controller video includes an assistor electronic device including an assistor display, and at least one user input device including at least a pointing device, the assistor electronic device programmed to perform a controller mirror task including receiving and displaying a mirror of the controller video on the assistor display and receiving remote inputs directed to the electronic imaging device controller via the at least one user input device of the assistor electronic device; and assistor electronic device interfacing hardware configured to transmit the controller video from the electronic imaging device controller to the assistor electronic device and including a human interface device (HID) restriction processor configured to receive the remote inputs from the assistor electronic device; transmit those remote inputs that satisfy an access criterion to the electronic imaging device controller; and block those remote inputs that do not satisfy the access criterion from the electronic imaging device controller.

In another aspect, an assistive method for providing remote assistance to a local operator of a medical imaging device having an electronic imaging device controller and a controller display presenting controller video includes at an assistor electronic device, receiving and displaying a mirror of the controller video and receiving remote inputs directed to the electronic imaging device controller via at least one user input device of the assistor electronic device; and using a HID restriction processor transmitting those remote inputs that satisfy an access criterion to the electronic imaging device controller; and blocking those remote inputs that do not satisfy the access criterion from the electronic imaging device controller.

In another aspect, an assistive method for providing remote assistance to a local operator of a medical imaging device having an electronic imaging device controller and a controller display presenting controller video includes an assistor electronic device including an assistor display, and at least one user input device including at least a keyboard, the assistor electronic device programmed to perform a controller mirror task including receiving and displaying a mirror of the controller video on the assistor display and receiving remote inputs directed to the electronic imaging device controller via the at least one user input device of the assistor electronic device, the remote inputs including at least inputs generated by the keyboard; and a HID restriction processor configured to receive the remote inputs from the assistor electronic device; transmit those remote inputs that satisfy an access criterion to the electronic imaging device controller; and block those remote inputs that do not satisfy the access criterion from the electronic imaging device controller.

One advantage resides in providing a remote expert with the ability to perform certain useful real-time control of the medical imaging device while preventing unintentional medical device interactions by the remote user during a remote imaging assistance session.

Another advantage resides in preventing a remote user from viewing sensitive data during a remote imaging assistance session.

Another advantage resides in preventing hacking of data during a remote imaging assistance session.

Another advantage resides in restricting a USB remote operation of a device to certain allowed actions by means of a hardware solution that is only controllable by the local side, thereby preventing a remote user from (unintended) accessing of certain information or functionality.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
Fig. 1 diagrammatically shows an illustrative apparatus for providing remote assistance in accordance with the present disclosure.
Figs. 2 and 3 show other aspects of the apparatus of Fig. 1.
Fig. 4 diagrammatically illustrates a nonlimiting example of a controller display, and Fig. 5 diagrammatically illustrates a corresponding display of a remote workstation showing a mirror of the controller display in a graphical user interface (GUI) window of a controller mirror task.

### DETAILED DESCRIPTION OF THE EMBODIMENTS

The following addresses security when enabling a Remote Operations Command Center (ROCC) to allow a remote expert to directly operate the medical imaging device controller of a medical imaging device during a medical imaging examination. There can be benefits to providing such remote control in enhancing the assistance that the remote expert can provided to the local imaging technologist, but patient safety considerations, government regulations, or the like may dictate that a remote user should not perform certain functions such as starting an imaging scan that applies radiation to the patient, operating robotic patient movement mechanisms, or so forth. Embodiments disclosed herein provide for beneficial remote control of the medical imaging device while blocking the remote user from performing certain operations that should not be done remotely.

In general, the remote workstation used by the remote expert performs a controller mirror task to provide a mirror of the display of the controller of the medical imaging device. The controller mirror task includes receiving and displaying controller video corresponding to the controller display on the remote workstation. In one approach for control display screen mirroring or sharing, the controller display is scraped by splitting the controller video signal to send to both the controller display and (via the Internet) to the remote expert where it is displayed using dedicated ROCC monitoring software. The remotely displayed (i.e., mirrored) controller video presented on the remote workstation suitably includes the pointer of a mouse or other pointing device shown on the controller display. In one approach, a keyboard, video, & mouse (KVM) switch is used at the local side to perform the video splitting.

In one approach for extending this to provide remote expert operation of the imaging controller, the remote expert software running on the remote expert's computer can capture the mouse pointer of the remote expert's computer when the mouse (or other pointing device) is focused on the window presenting the controller display. Alternatively, the mouse on the remote side is captured directly by a KVM receiver device. Thereafter, mouse interactions are sent (typically via the Internet) to the KVM switch at the local side which can then forward them to a USB port of the imaging controller to thereby allow the remote expert to control the mouse pointer of the imaging controller computer. In like fashion, control signals generated by other USB devices such as a USB keyboard can be sent to the KVM switch at the local side.

However, there are some challenges with this approach. The mouse movements are typically relative (Δx, Δy) movements under standard USB mouse protocol. Additionally, there may be a need to prevent the remote expert from performing prohibited actions such as starting a scan or robotically moving the patient. These issues are resolved as disclosed herein by a human interface device (HID) restriction processor that preprocesses and filters the USB input commands received from the remote expert.

To handle the relative (Δx,Δy) mouse movements, these are suitably converted to absolute movements based on the mouse sensitivity setting (which can be a configurable parameter of the HID restriction processor). To use this approach, the current pointer location is also used. This can be obtained in various ways, such as by maintaining a state machine at the HID restriction processor, or by analyzing the controller display video to extract the current pointer location.

To handle security issues, the remote USB inputs from the remote expert are filtered by restriction rules. If a restriction rule is violated then the violating USB input from the remote expert is not sent to the imaging controller, thereby preventing the violating USB input from being performed.

One complication to this approach for handling security issues is that some user inputs operate in combination. The relative (Δx,Δy) movements can be thought of as one example of this, i.e., the absolute mouse position is the integral or sum of the relative (Δx,Δy) movements. Another example is key combinations: For example, if one presses <CTRL> C, the usual process is the user presses and holds the control (<CTRL>) key and then presses and releases the "C" key and then finally releases the <CTRL> key. This will correspond to the following USB inputs (i.e., events) occurring overtime: (1) <CTRL> pressed; (2) "C" pressed; (3) "C" released; and (4) <CTRL> released. The <CTRL> C event is thus a combination of the <CTRL> key being pressed and not yet released together with the "C" key being pressed. To recognize such situations, the HID restriction processor can maintain a state machine storing the absolute position of the mouse pointer and the set of all keys currently being held down (i.e., that have been pressed but not yet released). Thus, for the <CTRL> C example, the restriction rule might be:

```
             IF input="C" pressed AND state(<CTRL>)=held THEN BLOCK input "C"
```

where state(<CTRL>)=held would be determined from the state machine maintained at the HID restriction processor.

Another complication to this approach for handling security issues is that the HID restriction processor only knows about inputs received from the remote expert, but not inputs received locally, for example inputs from the local imaging technologist via the mouse and/or keyboard of the imaging device controller. For example, suppose the last movement by the remote expert put the mouse pointer in a safe screen area where the remote expert is permitted to click on user interface (UI) elements. The state machine at the HID restriction processor will then indicate the mouse pointer of the imaging device controller is in this safe screen area. Thereafter, suppose the local user operates the local mouse to move the mouse pointer of the imaging device to a restricted screen area within which the remote user is not allowed to click. If the remote expert then clicks the remote mouse, if the local inputs are not recognized then the HID restriction processor will pass this click to the imaging device controller, because its state machine incorrectly indicates the mouse pointer is still in the safe screen area. This will result in a prohibited mouse click by the remote expert within the restricted screen area. Various approaches are disclosed herein to handle these types of situations.

In one approach, the HID restriction processor responds to any mouse click by the remote expert by first outputting a command to the imaging device controller to move the mouse pointer to the current absolute mouse pointer position as stored in the state machine of the HID restriction processor, before proceeding to process the mouse click by the remote expert as usual. This will ensure the mouse position of the imaging device controller matches that stored in the state machine of the HID restriction processor.

In another approach, the local user's mouse can be plugged into an additional USB port of the KVM switch at the local side, so that every USB mouse input from the local side is also received and processed by the HID restriction processor. In this case the absolute mouse pointer position of the state machine maintained by the HID restriction processor will correctly account for mouse movements by both the remote expert and the local user. In this case, the HID restriction processor would typically only apply the restriction rules to inputs received from the remote expert, but not to those inputs received from the local user. (In a variant embodiment, if the local user should also be restricted from performing certain inputs, then corresponding restriction rules could be applied to inputs received from the local user. For example, if the local user is a junior imaging technician who is not qualified to perform certain imaging operations, then these could be blocked.)

In yet another approach, if the HID restriction processor receives the controller display video (e.g., for the primary purpose of transmitting the controller display video to the remote workstation to achieve controller display mirroring at the remote workstation), and if the HID restriction processor has sufficient image processing capability, then the HID restriction processor can analyze a frame of the controller display video at the time of the mouse click to locate the mouse pointer in the controller display. This pointer location extracted from the controller display video frame can then be used for the processing of the mouse click. In this case there may be no need to maintain a state machine tracking the mouse pointer location.

In another variant embodiment, the HID restriction processor could be physically integrated with the KVM switch. This would enhance security since it would prevent anything from being inserted between the KVM switch and the HID restriction processor.

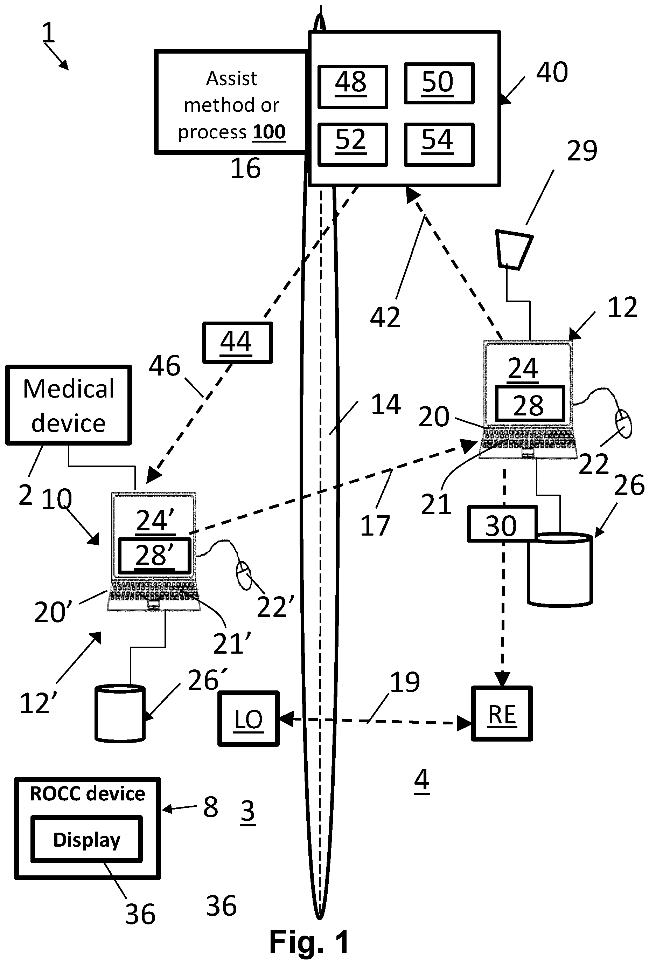

With reference to Fig. 1, an apparatus **1** for providing assistance from a remote medical imaging expert **RE** (or supertech) to a local technologist operator **LO** is shown. As shown in Fig. 1, the local operator **LO,** who operates a medical imaging device (also referred to as an image acquisition device, imaging device, and so forth) **2,** is located in a medical imaging device bay **3,** and the remote expert **RE** is disposed in a remote service location or center **4.** It should be noted that the "remote expert" **RE** may not necessarily directly operate the medical imaging device **2,** but rather provides assistance to the local operator **LO** in the form of advice, guidance, instructions, or the like. The remote location **4** can be a remote service center, a radiologist's office, a radiology department, and so forth. The remote location **4** may be in the same building as the medical imaging device bay **3** (this may , for example, in the case of a "remote operator or expert" **RE** who is a radiologist tasked with peri-examination image review), but more typically the remote service center **4** and the medical imaging device bay **3** are in different buildings, and indeed may be located in different cities, different countries, and/or different continents. In general, the remote location **4** is remote from the imaging device bay **3** in the sense that the remote expert **RE** cannot directly visually observe the imaging device **2** in the imaging device bay **3** (hence optionally providing a video feed as described further herein).

The image acquisition device **2** can be a Magnetic Resonance (MR) image acquisition device, a Computed Tomography (CT) image acquisition device; a positron emission tomography (PET) image acquisition device; a single photon emission computed tomography (SPECT) image acquisition device; an X-ray image acquisition device; an ultrasound (US) image acquisition device; or a medical imaging device of another modality. The imaging device **2** may also be a hybrid imaging device such as a PET/CT or SPECT/CT imaging system. While a single image acquisition device **2** is shown by way of illustration in Fig. 1, more typically a medical imaging laboratory will have multiple image acquisition devices, which may be of the same and/or different imaging modalities. Moreover, the remote service center **4** may provide service to multiple hospitals. The local operator controls the medical imaging device **2** via an electronic imaging device controller **10.** The remote operator is stationed at an assistance electronic device **12** (or, more generally, a remote workstation **12** or an electronic controller **12).**

Inputs from the electronic imaging device controller **10** can be sent to the remote electronic controller **12** via a communication link **14,** e.g., as a streaming video feed received via a secure Internet link.

The communication link **14** also provides a natural language communication pathway **19** for verbal and/or textual communication between the local operator and the remote operator. For example, the natural language communication link **19** may be a Voice-Over-Internet-Protocol (VOIP) telephonic connection, an online video chat link, a computerized instant messaging service, or so forth. Alternatively, the natural language communication pathway **19** may be provided by a dedicated communication link that is separate from the communication link **14,** e.g., the natural language communication pathway **19** may be provided via a landline telephone. In some embodiments, the natural language communication link **19** allows a local operator **LO** to call a selected remote expert **RE.** The call, as used herein, can refer to an audio call (e.g., a telephone call), a video call (e.g., a Skype or Facetime or other screen-sharing program), or an audio-video call. In another example, the natural language communication pathway **19** may be provided via a local electronic processing device, for example comprising an ROCC device **8,** such as a mobile device (e.g., a tablet computer or a smartphone), or can be a wearable device worn by the local operator **LO,** such as an augmented reality (AR) display device (e.g., AR goggles), a projector device, a heads-up display (HUD) device, etc., each of which having a display device **36.** For example, an "app" can run on the ROCC device **8** (operable by the local operator **LO)** and the assistance electronic device **12** (operable by the remote expert **RE)** to allow communication (e.g., audio chats, video chats, and so forth) between the local operator and the remote expert.

Fig. 1 also shows, in the remote service center **4** including the assistance electronic device **12,** such as an electronic processing device, a workstation computer, or more generally a computer, which is operatively connected to receive and present the video feed of the medical imaging device bay **3** from the camera **16** and/or to the audio feed from the microphone **15.** Additionally or alternatively, the assistance electronic device **12** can be embodied as a server computer or a plurality of server computers, e.g., interconnected to form a server cluster, cloud computing resource, or so forth. The workstation **12** includes typical components, such as an electronic processor **20** (e.g., a microprocessor), at least one user input device, typically including a keyboard **21** and at least one pointing device **22,** such as an illustrative mouse **22,** or a trackball, trackpad, touch-sensitive display, or so forth. The workstation **12** further includes at least one display device **24** (e.g., an LCD display, plasma display, and/or so forth). In some embodiments, the display device **24** can be a separate component from the workstation **12.** The display device **24** may also comprise two or more display devices. The electronic processor **20** is operatively connected with a one or more non-transitory storage media **26.** The non-transitory storage media **26** may, by way of non-limiting illustrative example, include one or more of a magnetic disk, RAID, or other magnetic storage medium; a solid state drive, flash drive, electronically erasable read-only memory (EEROM) or other electronic memory; an optical disk or other optical storage; various combinations thereof; or so forth; and may be for example a network storage, an internal hard drive of the workstation **12,** various combinations thereof, or so forth. It is to be understood that any reference to a non-transitory medium or media **26** herein is to be broadly construed as encompassing a single medium or multiple media of the same or different types. Likewise, the electronic processor **20** may be embodied as a single electronic processor or as two or more electronic processors. The non-transitory storage media **26** stores instructions executable by the at least one electronic processor **20.** The instructions include instructions to generate a graphical user interface (GUI) **28** for display on the remote operator display device **24.** The video feed from the camera **16** can also be displayed on the display device **24,** and the audio feed from the microphone **15** can be output on the assistance electronic device **12** via a loudspeaker **29.** In some examples, the audio feed can be an audio component of an audio/video feed (such as, for example, recording as a video cassette recorder (VCR) device would operate). To provide the controller display mirroring functionality at the remote workstation **12,** the remote workstation **12** is programmed to perform a controller mirror task under the GUI **28,** including receiving and displaying the controller video **17** on the assistor display and receiving remote inputs from the user input devices **21** and **22** that are directed to the electronic imaging device controller **10** via the at least one user input device **21, 22** of the assistor electronic device **12.**

The medical imaging device controller **10** in the medical imaging device bay 3 also includes similar components as the assistance electronic device **12** disposed in the remote service center **4.** Except as otherwise indicated herein, features of the medical imaging device controller **10,** which includes a local workstation **12',** disposed in the medical imaging device bay **3** similar to those of the assistance electronic device **12** disposed in the remote service center **4** have a common reference number followed by a "prime" symbol, and the description of the components of the medical imaging device controller **10** will not be repeated. In particular, the medical imaging device controller **10** typically includes a keyboard **21'** and at least one pointing device **22',** such as an illustrative mouse **22',** or a trackball, trackpad, touch-sensitive display, or so forth. The medical imaging device controller **10** is configured to display a GUI **28'** on a display device or controller display **24'** that presents information pertaining to the control of the medical imaging device **2,** such as configuration displays for adjusting configuration settings an alert **30** perceptible at the remote location when the status information on the medical imaging examination satisfies an alert criterion of the imaging device **2,** imaging acquisition monitoring information, presentation of acquired medical images, and so forth. It will be appreciated that the controller display screen mirroring data stream (i.e., controller display video) **17** carries the content presented on the display device **24'** of the medical imaging device controller **10.** The communication link **14** allows for screen sharing of the controller display video **17** from the controller **10** to the remote workstation **12.** The GUI **28'** running on the medical imaging device controller **10** includes one or more dialog screens, including, for example, an examination/scan selection dialog screen, a scan settings dialog screen, an acquisition monitoring dialog screen, among others. The GUI **28'** can be included in the video feed **17** and displayed on the assistor electronic device display **24** of the remote workstation (i.e., assistor electronic device) **12** at the remote location **4.**

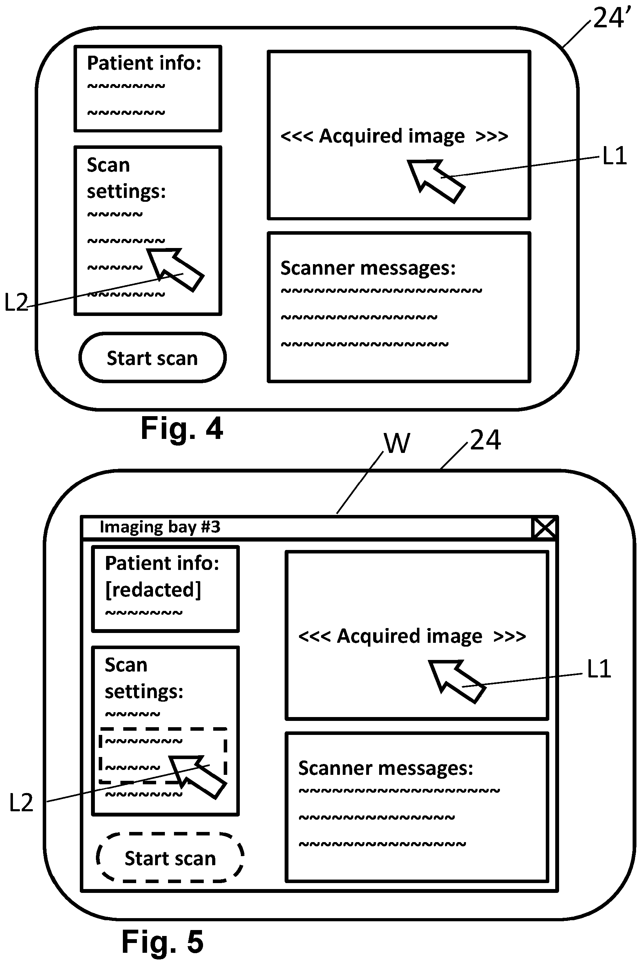

The electronic device **12** is programmed to perform a controller mirror task that includes receiving and displaying the controller video **17** on the assistor display **24** and receiving remote inputs from the user input devices **21** and **22** of the assistor device **12** that are directed to the electronic imaging device controller **10.** The controller mirror task running on the assistor electronic device **12** may be a software program or the like which presents the controller video **17,** possibly in modified form, in a window **W** (see Fig. 5) of the GUI **28.** In a suitable embodiment, the GUI **28** is implemented by a windowing operating system of the assistor electronic device **12.** For example, the assistor electronic device **12** may be running a Microsoft Windows operating system (available from Microsoft Corporation, Redmond, WA, USA), or may be running a MacOS operating system (available from Apple Corporation, Cupertino, CA, USA), or may be running a GNU/Linux distribution or BSD operating system (numerous variants of which are available from various commercial software vendors and free software distributors), as some nonlimiting illustrative examples. In a typical GUI-based windowing operating system such as Windows, MacOS, GNU/Linux, or BSD, tasks running under the operating system are assigned windows in the GUI. The user of such a windowing operating system can switch between different windows (and thus corresponding tasks) by various approaches. The window/task the user is currently using is often referred to as the window/task that has focus, and user inputs from the user input devices **21, 22** are received by the window/task that currently has focus. For example, in some approaches, a user may click in a window to focus on that window/task. Other approaches can be used to move the focus to a particular window/task, such as a special key combination for switching between windows/tasks (e.g., <ALT>+<TAB> in some operating systems). Of interest here, when the user of the assistor electronic device **12** moves focus to the window **W** presenting the controller display video **17** in accordance with the controller mirror task, thereafter any inputs made by the user via a user input device such as the keyboard **21** or mouse **22** are directed to the controller mirror task unless and until the user switches focus to another task running on the assistor electronic device **12.** For example, if the user has focused the GUI **28** on the window **W** presenting the controller video **17,** then inputs made via the user input devices **21, 22** constitute remote inputs directed to the controller mirror task and hence directed to the electronic imaging device controller **10** as disclosed herein.

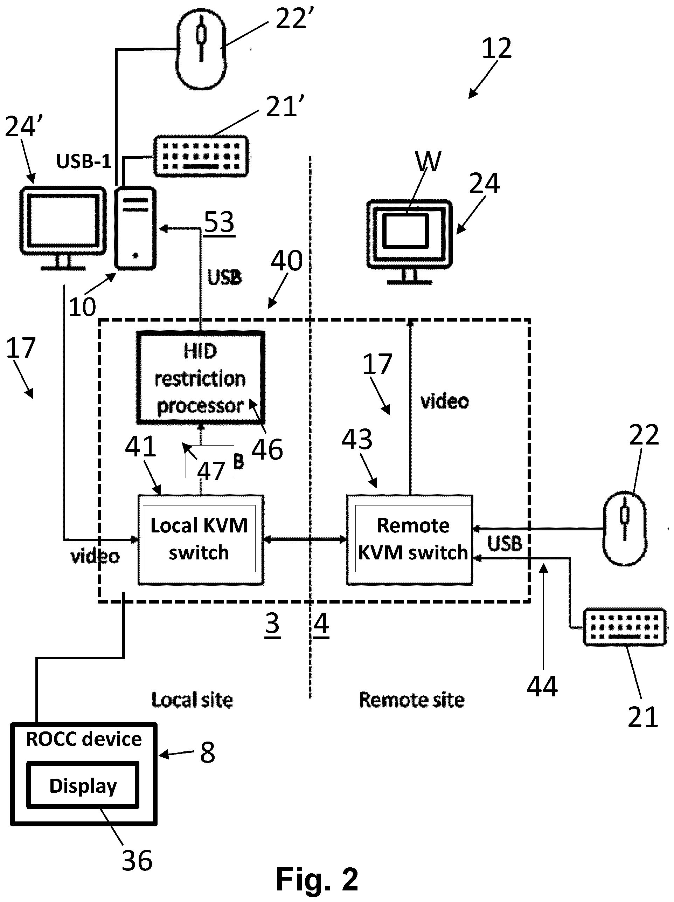

With reference to Fig. 2, and with continuing reference to Fig. 1, the apparatus **1** includes assistor electronic device interfacing hardware **40** (diagrammatically shown as a rectangle in Fig. 1) that provides for interfacing between the electronic imaging device controller **(10)** and the remote workstation **12** (or, more generally, the assistor electronic device **12).** In the illustrative example, the assistor electronic device interfacing hardware **40** comprises a keyboard, video, monitor (KVM) switch including a local KVM switch **41** connected to the medical device imaging controller **10** via a USB connection **42,** and a remote KVM switch **43** connected to the remote workstation **12** via a different USB connection **44.** The local KVM switch **41** operates to transmit the controller video **17** to the remote KVM switch **43** and to receive remote user inputs from the remote KVM switch **43;** and conversely the remote KVM switch **43** operates to receive the controller video **17** from the local KVM switch **41** and to transmit remote user inputs to the local KVM switch **41.** In other examples, the assistor electronic device interfacing hardware **40** may be constructed of a set of discrete video signal splitter and combiner components and discrete USB signal transmitters and receivers. The local KVM switch **41** and the remote KVM switch **43** intercommunicate via the communication link **14** (e.g., comprising the Internet). The controller video **17** is thereby output to the assistor electronic device **12,** where it is displayed on the display device **24** via the GUI **28** (e.g. via the controller mirror task running on the assistor electronic device **12)** to allow the assistor (e.g. the ROCC remote expert **RE)** to view the controller video **17** displayed on the assistor electronic device **12.** The assistor electronic device **12** used by the remote expert **RE** is also programmed to provide remote inputs received via the at least one user input device **21, 22** and directed to the electronic imaging device controller **10** (for example, due to the inputs from the at least one user input device **21, 22** being received while the GUI **28** is focused on the controller mirroring task). Alternatively, the controller display video **17** may be displayed by the KVM switch **43** directly on a screen (not shown) connected thereto, while mouse **21'** and keyboard **22'** for remote operation are also connected directly to the KVM switch **43.**
The assistor electronic device interfacing hardware **40** includes a Human Interface Device (HID) restriction processor **46** programmed to control the transmission of remote inputs **47** from the user input devices **21, 22** of the remote workstation **24** to the imaging device controller **10.**

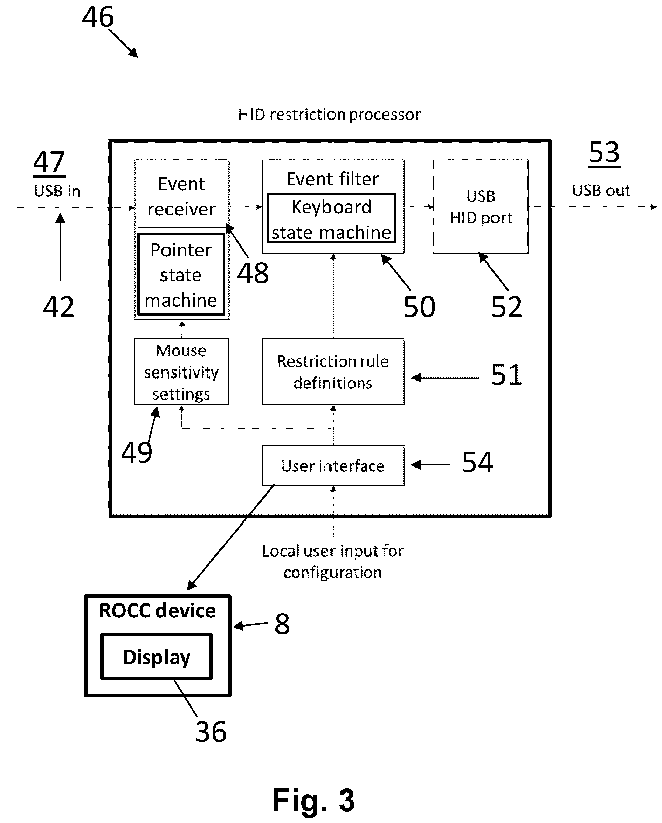

With continuing reference to FigS. 1 and 2, Fig. 3 shows an embodiment of the HID restriction processor **46.** As shown in Fig. 3, the remote inputs **47** received from the user input devices **21, 22** of the remote workstation **24** are received via the remote KVM switch **43** and local KVM switch **41** (see Fig. 2). The HID restriction processor **46** then acts as a gateway to transmit those remote inputs that satisfy an access criterion to the electronic imaging device controller **10,** while blocking those remote inputs that do not satisfy the access criterion from the electronic imaging device controller **10.** The illustrative user input devices **21, 22** of the remote workstation **24** are USB devices (e.g., an illustrative USB keyboard **21** and USB mouse **22),** and hence the illustrative remote inputs **47** are USB signals. However, in other embodiments it is contemplated for the remote inputs to be in another data format. The remote inputs **47** are transmitted to an event receiver and absolute mouse coordinate translation module **48** (i.e., an "event receiver" module **48)** that format conversion, translation from relative to absolute coordinates, and/or other preprocessing of the remote inputs **47.** The event receiver **48** is configured to allow all keyboard events from the remote keyboard **21** to pass through without modification, but preprocesses the mouse events from the remote mouse **22** if they are in relative coordinates. USB mouse events are mostly transmitted as relative translation messages (i.e., the message contains the Δ in x and y directions that the remote mouse **22** was moved. In this case, the receiver event receiver **48** is configured to interpret these messages in the context of the screen resolution and translate the relative coordinates into the absolute coordinates of the mouse cursor of the imaging device controller **10.** The event receiver **48** is configured to integrate the translation steps (i.e., the relative coordinates Δx and Δy) to absolute coordinates, thereby translating every relative mouse motion to an absolute mouse position, and then send the respective absolute positioning USB message instead. In addition to integrating the relative movements Δx and Δy, this conversion from relative pointer coordinates to absolute pointer coordinates relies on knowledge of the current location of the pointer of the imaging device controller **10.** In one approach for determining the current location of the pointer, the event receiver **48** maintains a pointer state machine that stores the current location of the pointer. Hence, for example, if the pointer state machine stores the current pointer location as (xᵢ,yᵢ) and the remote input provides a relative movement of (Δx, Δy), then the event receiver converts this relative movement to absolute coordinates (xᵢ+Δx,yᵢ+Δy). To enable the pointer state machine to accurately account for movements of the on-screen pointer made by the local user via the local mouse **22',** the local mouse inputs could also be applied to the event receiver **48,** although these local mouse inputs would only be used for updating the pointer state machine and would not be filtered by the event filter **50.**
Other approaches can be used to determine the current location of the mouse pointer, such as extracting it from image processing of a frame of the controller video **17** corresponding in time to the relative movement of (Δx, Δy). In this case, the controller video **17** would need to be an input to the event receiver **48** and the event receiver **48** would need to have sufficient image processing capability to, for example, apply a matched filter to the controller video frame to locate the mouse pointer. The event receiver **48** may also be supplied with mouse sensitivity settings **49** controllable by the local operator **22',** which specify the weighting coefficients for x and y translations when summing them to absolute positions. The mouse sensitivity settings **49** store the mouse sensitivity coefficients defined by the local operator **LO** or by a technician setting up and configuring the apparatus **1.** The foregoing assumes that the remote USB mouse input is in relative coordinates. If, on the other hand, the incoming remote USB mouse input is already in absolute positioning format, they will be passed on through the event receiver **48** unchanged.

An event filter module **50** is configured to compare the incoming keyboard and mouse events to a table of restriction definitions. If an incoming event is not restricted, it will be passed on unchanged. If an incoming event is restricted, it will be rejected and not be passed on. For example, the event filter module **50** is configured to implement a set of restriction rule definitions **51** that contains a list (or table) of restriction rules to decide which keyboard or mouse events are allowed or prohibited. Restriction definitions include, but are not limited to: Individual keys (such as the "Windows" key or function keys), Key combinations / shortcuts (such as Ctrl-^{∗} or Alt-^{∗} combinations), Mouse positions (areas), Mouse clicks in defined areas, and so forth. The restriction definitions **51** depend on the system type and the system GUI and need to be defined specifically for each imaging device **2.** Example configurations include, but are not limited to: "allow only mouse movement, no clicks, no keyboard (no absolute coordinate translation required);" "allow only letters, numbers, shift, but no other keys; allow mouse clicks except for restricted areas (such as scan start button)," and so forth. To deal with multiple key combinations, the event filter **50** may include a keyboard state machine, the purpose of which is as follows. The USB keyboard generally generates one USB event when a key is pressed, and a second USB event when that key is released. If, for example, the event filter **50** is to filter out a key combination such as ALT-X, then this key combination will typically include the events: "ALT" pressed, then "X" pressed, then "X" released, then "ALT" released. The keyboard state machine suitably tracks which keys are currently pressed (and not yet released), so that a key combination such as ALT-X can thereby be detected. Hence, in this example to block the ALT-X combination the remote input consisting of "X" being pressed is passed through the filter **50** unless the keyboard state machine indicates the "ALT" key is also currently pressed (and not yet released), in which case the "X" pressed remote input is blocked by the filter **50.**

The HID restriction processor **46** further includes a USB port **52** that allows the HIS restriction processor **46** to make itself discoverable as a USB device. When the USB port **52** is connected to a USB port or hub of the imaging device controller **10,** filtered remote USB inputs **53** are output via the USB port **52** as input to the imaging device controller **10.** Hence, the imaging device controller **10** receives the remote inputs **47** provided by the assistor electronic device **12,** but filtered by the event filter **50** in accordance with the restriction rule definitions **51** so that the user of the remote assistor device **12** is thereby enabled to remotely control the imaging device controller **10,** but only as restricted by the HID restriction processor **46.**

The HID restriction processor **46** optionally provides a user interface **54** (for example, a web interface, or a data transmission port with pre-defined protocol) onto the display device **36** of the ROCC device **8,** where the local operator **8** can change the restriction rule definitions **51** and the mouse sensitivity settings **49.** Some (or all) definitions **51** and settings **49** may also fixed in the ROCC device **8** firmware without the option for user modifications. With reference to Figs. 4 and 5, operation of the system is described by way of an illustrative example. Fig. **4** diagrammatically shows the controller display presented on the controller display device **24'.** This illustrative display is suitably shown during an image acquisition scan, and includes screen regions or windows presenting: "Patient info" containing information about the patient being imaged, "Scan settings" containing the current scan settings, a "Start scan" button operable to start the imaging scan, an "<<<Acquired image>>>" region which presents the last-acquired image of the patient, and a "Scanner messages" region that presents information generated by the imaging device in human-readable format. Under local operation, the local user can operate the mouse **22'** (or other pointing device) to move an on-screen pointer to a location chosen by the local user, for example to an illustrative location **L1** in the "<<<Acquired image>>>" region, or to an illustrative location **L2** in the "Scan settings" region. (It will be appreciated that at any given time the pointer will be at only one location, but the two locations **L1** and **L2** are shown in Figs. 4 and 5 for explanatory purposes). The local user can generally click anywhere on the screen and have the effect of that click, for example the local user could move the mouse pointer to the **L2** location and click to modify a scan setting.

The remote expert **RE** operating the assistor electronic device **12** can also perform remote operations, but limited by the HID restriction processor **46.** More particularly, Fig. 5 shows the mirrored controller display in a window **W** shown on the display device **24** of the assistor electronic device **12.** The window **W** is shown occupying a large portion, but not all, of the screen space of the assistor display **24.** However, as is known in the art, a typical GUI-based windowing operating system (e.g., Microsoft Windows, MacOS, the desktop environment of a GNU/Linux distribution, or so forth) typically allows for windows to be resized, expanded to occupy the entire screen, or otherwise manipulated, so the window **W** shown in Fig. 5 is only an illustrative example. Alternatively, the mirrored controller display is shown via a direct connection of a KVM receiver device to a screen. As seen in Fig. 5, the controller display mirrored in the window **W** is for the most part a verbatim copy of the content of the controller display **24'** as shown in Fig. 4. However, it is contemplated for some content to be redacted, e.g., as indicated by the "[redacted]" indication in the "Patient info" region of the mirrored display shown in Fig. 5. Such optional redaction, if done, can be suitably implemented by image processing of video frames of the controller video **17** performed at the local KVM switch **41,** for example. In general, the remote user can also perform control events, such as keyboard presses or mouse clicks, anywhere in the mirrored controller display shown in the window **W,** and those become remote inputs **47** to the imaging device controller **10.** However, as previously described, the HID restriction processor **46** filters these remote inputs **47,** and those remote inputs that satisfy the access criterion (e.g., as specified by the restriction rule definitions **51,** see Fig. 3) are transmitted to the electronic imaging device controller **10** while any remote inputs that do not satisfy the access criterion are blocked from the electronic imaging device controller **10** (for, example, being blocked by the event filter **50** not sending those remote inputs on to the USB HID port **52).**

As diagrammatically shown in Fig. 5, those areas where the remote user is not allowed to click are indicated by dashed boxes - these include some (but not all) of the settings in the "Scan settings" region, and the "Start scan" button. Hence, if the pointer is at the **L1** location the remote user can perform operations such as clicking and holding the mouse to manipulate the image shown in the "<<<Acquired image>>> region. However, if the pointer is at the **L2** location the remote user is blocked by the HID restriction processor **46** from performing operations such as clicking to change a scan setting. Optionally, the controller mirror task presenting the mirrored controller display in the window **W** shown on the assistor display **24** may modify the mirrored content to indicate regions where the operator of the assistor electronic device **12** is not allowed to access functions. For example, the dashed boxes diagrammatically shown in Fig. 5 could actually be shown superimposed on the mirrored controller display, and/or the mouse pointer shown on the mirrored display could change color or otherwise be visually modified when it is in a restricted area (e.g., this is shown by hatching of the mouse pointer at the location **L2** in Fig. 5). By such restrictions, the remote user may be blocked, for example, from starting a scan, accessing certain menu items, changing the view, and/or so forth. This can be realized by either restricting the mouse cursor to prevent the remote user from moving the mouse pointer into these areas (in this case, the remote user would be blocked from even moving the mouse pointer to the location **L2),** or by filtering out the mouse clicks while the cursor is in the defined areas (e.g., when the mouse pointer is at the location **L2** in Fig. 5). In addition, access of these menu items and scan start via keyboard could be restricted by filtering out any Alt+^{∗} key combinations, all Ctrl+Alt+^{∗} combinations, and all function keys, as previously described.

The HID restriction processor **46** is configured to determine whether inputs (e.g., mouse movements or keyboard keystrokes) provided to the controller video **17** satisfy a predetermined access criterion to determine whether the assistor can have access to the ROCC device **8** operable by the local operator **LO.** The HID restriction processor **46** is configured to process the controller video **17** and input the processed controller video **17** to the local KVM switch **41** which transmits the video **17** to the remote KVM switch **43.** In some embodiments, the HID restriction processor **46** is integrated with the local KVM switch **41.**

Furthermore, as disclosed herein, the ROCC device **8,** the assistor electronic device **12,** and the assistor electronic device interfacing hardware **40** are configured to perform a method or process **100** for providing assistance during a medical imaging examination performed using a medical imaging device **2** (i.e., by assisting local operators **LO** of respective medical imaging devices **2** during medical imaging examinations by a remote expert **RE).** The instructions to perform the method **100** are stored in the non-transitory computer readable medium **26** of the assistance electronic device **12,** in the assistor electronic device interfacing hardware **40,** and in the ROCC device **8.**

In one embodiment, the restriction definitions **51** are pre-set for different devices. It is then only necessary to set the device type to populate the list of restrictions accordingly.

In one embodiment, a set of restriction definitions **51** is defined per user privileges or user role. Depending on the local and/or remote user, different sets of restrictions are applied. Identification of the local/remote user can be done manually (selectable by local user) or automatically (requires an additional connection of the HID restriction processor **46** to a central management system of the virtualization solution, such as the ROCC device **8).**

In one embodiment, certain or all restrictions can be released temporarily by the local user by pushing a button (i.e., on the ROCC device **8).**

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. An assistive system **(1)** for providing remote assistance to a local operator **(LO)** of a medical imaging device **(2)** having an electronic imaging device controller **(10)** and a controller display **(24')** presenting controller video **(17),** the assistive system comprising:
an assistor electronic device **(12)** including an assistor display **(24),** and at least one user input device **(21, 22)** including at least a pointing device **(22),** the assistor electronic device programmed to perform a controller mirror task including receiving and displaying a mirror of the controller video on the assistor display and receiving remote inputs directed to the electronic imaging device controller via the at least one user input device of the assistor electronic device; and
assistor electronic device interfacing hardware **(40)** configured to transmit the controller video from the electronic imaging device controller to the assistor electronic device and including a human interface device (HID) restriction processor **(46)** configured to:
receive the remote inputs from the assistor electronic device;
transmit those remote inputs that satisfy an access criterion **(51)** to the electronic imaging device controller; and
block those remote inputs that do not satisfy the access criterion from the electronic imaging device controller.

2. The assistive system **(1)** of claim 1, wherein the remote inputs include a pointer movement input generated by the pointing device **(22)** of the assistor electronic device **(12),** and the HID restriction processor **(46)** is configured to:
transmit the pointer movement input to the electronic imaging device controller **(10)** if the pointer movement input would place an on-screen pointer of the electronic imaging device controller in an allowed region of the controller display **(24')** as defined by the access criterion **(51);** and
block the pointer movement input from the electronic imaging device controller if the pointer movement input would place the on-screen pointer of the electronic imaging device controller into a restricted area as defined by the access criterion.

3. The assistive system **(1)** of claim 2, wherein the pointer movement input is in relative coordinates and the HID restriction processor **(46)** is further configured to convert the pointer movement input from the relative coordinates to absolute coordinates based on a current location of the on-screen pointer of the imaging device controller **(10).**

4. The assistive system **(1)** of claim 3, wherein HID restriction processor **(46)** is configured to convert the pointer movement input from the relative coordinates to absolute coordinates by:
summing different movements of the mouse cursor performed by the pointing device **(22)** of the assistor electronic device **(12);** and
determining the absolute measurement as the current location of the on-screen pointer of the electronic imaging device controller **(10)** plus the sum of the different movements of the mouse cursor.

5. The assistive system **(1)** of any one of claims 1-4, wherein the remote inputs include a button click input generated by the pointing device **(22),** and the HID restriction processor **(46)** is configured to:
transmit the button click input to the electronic imaging device controller **(10)** if a current location of the on-screen pointer of the imaging device controller is in an allowed region of the controller display **(24')** as defined by the access criterion **(51);** and
block the button click input from the electronic imaging device controller **(10)** if the current location of the on-screen pointer of the imaging device controller is in a restricted region of the controller display **(24')** as defined by the access criterion **(51).**

6. The assistive system **(1)** of any one of claims 1-5, wherein the at least one user input device **(21, 22)** further includes a keyboard **(21)** of the assistor electronic device **(12),** the remote inputs include a keyboard input generated by the keyboard of the assistor electronic device, and the HID restriction processor **(46)** is configured to:
transmit the keyboard input to the electronic imaging device controller **(10)** if the keyboard input satisfies the access criterion **(51);** and
block the keyboard input from the electronic imaging device controller **(10)** if the keyboard input does not satisfy the access criterion **(51).**

7. The assistive system **(1)** of any one of claims 1-6, wherein the mirror of the controller video displayed at the assistor electronic device has redactions.

8. The assistive system **(1)** of any one of claims 1-7, further comprising:
a local electronic device **(8)** operable by the local operator **(LO)** and operatively connected to provide a user interface **(54)** for configuring settings of the HID restriction processor **(46).**

9. The assistive system **(1)** of any one of claims 1-10, wherein the assistor electronic device interfacing hardware **(40)** further includes at least one keyboard, video, monitor (KVM) switch **(41, 43).**

10. The assistive system **(1)** of claim 9, wherein the at least one KVM switch includes:
a local KVM switch **(41)** connected with or integral with the HID restriction processor **(46);** and
a remote KVM switch **(43)** connected with the assistor electronic device **(12);**
the local KVM switch and the remote KVM switch connected to intercommunicate via the Internet.

11. An assistive method **(100)** for providing remote assistance to a local operator **(LO)** of a medical imaging device **(2)** having an electronic imaging device controller **(10)** and a controller display **(24')** presenting controller video **(17),** the assistive system comprising:
at an assistor electronic device **(12),** receiving and displaying a mirror of the controller video and receiving remote inputs directed to the electronic imaging device controller via at least one user input device **(21, 22)** of the assistor electronic device; and
using a human interface device (HID) restriction processor **(46):**
transmitting those remote inputs that satisfy an access criterion **(51)** to the electronic imaging device controller; and
blocking those remote inputs that do not satisfy the access criterion from the electronic imaging device controller.

12. The assistive method **(100)** of claim 11, wherein the remote inputs include a pointer movement input generated by a pointing device **(22)** of the assistor electronic device **(12),** wherein the transmitting and blocking include:
transmitting the pointer movement input to the electronic imaging device controller **(10)** if the pointer movement input would place an on-screen pointer of the electronic imaging device controller in an allowed region of the controller display **(24')** as defined by the access criterion **(51);** and
blocking the pointer movement input from the electronic imaging device controller if the pointer movement input would place the on-screen pointer of the electronic imaging device controller into a restricted area as defined by the access criterion.

13. The assistive method **(100)** of claim 12, wherein the pointer movement input is in relative coordinates and method further includes converting the pointer movement input from the relative coordinates to absolute coordinates based on a current location of the on-screen pointer of the imaging device controller.

14. The assistive method **(100)** of claim 13, wherein the converting includes:
summing different movements of the mouse cursor performed by the local operator **(LO)** and the assistor; and
determining the absolute measurement as the current location of the on-screen pointer of the electronic imaging device controller **(10)** plus the sum of the different movements of the mouse cursor.

15. The assistive method **(1)** of any one of claims 11-14, wherein the remote inputs include a button click input generated by a pointing device **(22)** of the assistor electronic device **(12),** wherein the transmitting and blocking include:
transmitting the button click input to the electronic imaging device controller **(10)** if a current location of the on-screen pointer of the imaging device controller is in an allowed region of the controller display **(24')** as defined by the access criterion **(51);** and
blocking the button click input from the electronic imaging device controller **(10)** if the current location of the on-screen pointer of the imaging device controller is in a restricted region of the controller display **(24')** as defined by the access criterion **(51).**
